# EUROPEAN PATENT SPECIFICATION

(11) **EP 3 731 828 B1**
(45) Date of publication and mention of the grant of the patent: **02.12.2026**
(21) Application number: 18918420.3
(22) Date of filing: 27.12.2018
(51) Int. Cl.: A61K 9/24, A61K 9/28, A61K 31/00

(54) **TABLET FORMULATIONS COMPRISING METFORMIN AND SITAGLIPTIN PROCESSED WITH HOT-MELT EXTRUSION**
DURCH HEISSSCHMELZEXTRUSION VERARBEITETE TABLETTENFORMULIERUNGEN MIT METFORMIN UND SITAGLIPITIN
FORMULATIONS DE COMPRIMÉS COMPRENANT DE LA METFORMINE ET DE LA SITAGLIPTINE TRAITÉES PAR EXTRUSION À CHAUD

(30) Priority: 28.12.2017 TR 201722603
(43) Date of publication of application: 04.11.2020
(73) Proprietor: Sanovel Ilac Sanayi ve Ticaret A.S., Sisli - Istanbul (TR)
(72) Inventor: TÜRKYILMAZ, Ali, 34460 Istanbul (TR); ZENGINER, Sibel, 34460 Istanbul (TR); ATAMAN, Seval, 34460 Istanbul (TR)
(74) Representative: Genç Ilhan, Oznur
(86) International application number: PCT/TR2018/050918
(87) International publication number: WO 2019/240699

(56) References cited:
- WO-A1-2009/111200
- WO-A2-2007/041053
- WO-A2-2020/046243
- US-A1- 2008 064 701
- PRATHIMA SRINIVAS M ET AL: "Formulation and evaluation of sitagliptin phosphate and metformin hydrochloride trilayered tablets", INTERNATIONAL JOURNAL OF DRUG DELIVERY, ADVANCED RESEARCH JOURNALS, US, vol. 5, no. 1, 1 January 2013 (2013-01-01), pages 15 - 27, XP009179090, ISSN: 0975-0215

## Description

### Field of the invention

The present invention relates to pharmaceutical tablet formulations comprising metformin and sitagliptin and at least one pharmaceutically acceptable excipient, as defined by the claims, which is prepared by a hot-melt extrusion method for the preparation of said composition.

### Background of the invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2:
Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose.

In Type 2 diabetes, the pancreas makes insulin, but it either doesn't produce enough, or the insulin does not work properly. This diabetes occurs most often in people who are over 40 years old and overweight. Type 2 diabetes may sometimes be controlled with a combination of diet, weight management, and exercise. However, treatment also may include oral glucose-lowering medications or insulin injections.

Metformin is antidiabetics having an orally-administrated biguanide structure. Metformin hydrochloride is a white to off-white crystalline compound and it is freely soluble in water and practically insoluble in acetone, ether and chloroform. Oral doses of metformin are generally recommended in the range of 500 to 2500 mg a day and a single dose may vary from 250 to 1000mg. It is used singly or in combination with sulfonylureas, alpha-glucosidase inhibitors, or insulin.

The chemical name of metformin hydrochloride is 1,1-dimethylbiguanide hydrochloride, has the following chemical structure of Formula I.

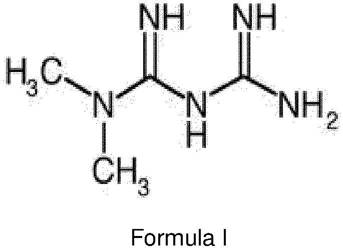

Although metformin is effective at lowering blood glucose levels, its use is associated with gastrointestinal (GI) adverse effects, particularly diarrhea and nausea. These adverse effects may limit the tolerated dose of metformin and cause patients to discontinue the therapy.

Sitagliptin is indicated as an adjunct to diet and exercise to improve glycemic control in adults with type 2 diabetes mellitus. It is an oral antihyperglycemic of the dipeptidyl peptidase-4 (DPP-4) inhibitor class. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Sitagliptin works by binding to DPP-4 and preventing it from breaking down the GLP-1 and GIP. This increases the levels of these hormones in the body and so increases their effect on controlling blood sugar.

The chemical name of sitagliptin is (3R)-3-amino-1-[3-(trifluoromethyl)-6,8-dihydro-5H-[1,2,4]triazolo[4,3-a]pyrazin-7-yl]-4-(2,4,5-trifluorophenyl) butan-1-one or (2R)-4-oxo-4- [3-(trifluoromethyl)-5,6-dihydro[1,2,4]triazolo[4,3-a]pyrazin-7(8H)-yl]-1-(2,4,5trifluorophenyl) butan-2-amine) and its chemical structure is shown in the Formula II. W2

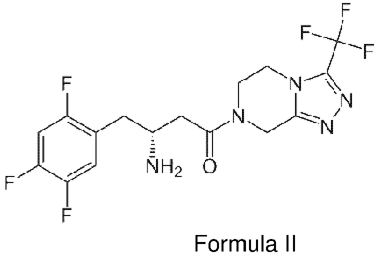

Sitagliptin is disclosed in the patent US6699871. A crystal phosphate monohydrate form of sitagliptin is disclosed in the patent WO2005003135.

DPP-4 inhibitors, especially sitagliptin have a novel mechanism of action and many studies are available which shows their efficacy and safety in monotherapy or combination therapy. The single sitagliptin alone does not provide adequate glycemic control.

Sitagliptin increases plasma GLP-1 concentration and elevates cellular cAMP levels in pancreatic beta-cells leading to potentiate insulin secretion, whereas metformin improves glucose tolerance in patients with Type 2 diabetes, lowering both basal and postprandial plasma glucose. Its pharmacologic mechanisms of action are different from other classes of oral antihyperglycemic agents in that metformin decreases hepatic glucose production, decreases intestinal absorption of glucose and improves insulin sensitivity by increasing peripheral glucose uptake and utilization. Therefore, they both help to reduce blood glucose levels in different pathways in type 2 diabetes patients.

A tablet formulation for metformin and sitagliptin combination is commercially available under the trade name Janumet^{®}. It contains either 500 or 1000 mg metformin, and 50 mg sitagliptin. Pharmaceutical compositions comprising fixed-dose combinations of immediate-release sitagliptin and metformin are disclosed in WO 2007/078726 patent application which published on July 12, 2007.

U.S. publication number 2012/0202820 discloses pharmaceutical compositions comprising sitagliptin and metformin in combination with a lubricant, wherein the lubricant is polyethylene glycol or mixtures of polyethylene glycol with one or more other lubricants and comprises more than 10% by weight of the total weight of the composition.

Sitagliptin and metformin combination has incompatibility and stability problems due to interactions between drug substances. In the present invention, multi-layered tablet in which Sitagliptin and metformin are in different layers is formulated so as to overcome the problems. Thus, not only metformin and sitagliptin did not interact with each other, but also this provides easy and cost-effective process.

In the present invention, it has been surprisingly observed that an unexpected and also a synergistic therapeutic benefit can be obtained in the treatment of type-2 diabetes with a combination therapy comprising metformin and sitagliptin, provided with minimizing problem of incompatibility.

WO 2009/111200 A1 discloses a tablet formulations comprising a core having metformin coated by a first layer of sustained release polymer separating it from a second layer comprising sitagliptin. US 2008/064701 A discloses a tablet core comprising metformin and povidone prepared by wet granulation, subsequently seal coated with Opadry and further coated with a layer of sitagliptin. Said documents does not propose any solution for an improved homogeneity. Enhanced dissolution of the active agents and desired uniformity in the content are not linked with the amount of any of the core or layer formulation.

XP009179090 discloses a trilayered tablet formulation comprising metformin and sitagliptin as active agents. Actually, no separation layer is disclosed between the metformin containing portion (core) and sitagliptin containing layer.

US 2008/064701 A1 discloses tablet cores comprising metformin and povidone (dispersion carrier) prepared by wet granulation methods, subsequently seal coated with e.g. Opadry and further coated with a layer of sitagliptin (ex.1-2).

The combination preparations described in the prior art have the drawback that the tablets can only be produced by wet granulation, as the active substance metformin is a very poorly compressible active substance. In wet granulation process there is, however, the risk that the active substance might be decomposed through interactions with the solvent used, that causes formation of undesirable degradation products.

Therefore, there is also still a need for a pharmaceutical tablet formulation comprising metformin and sitagliptin, having desired stability.

### Detailed description of the Invention

The main object of the present invention is to avoid incompatibility problems between metformin and sitagliptin using multi-layered tablet form.

Another object of the present invention is to provide both stability and content uniformity of metformin in the core.

The term "core" as used herein refers to a compact mass having a definite geometric shape such as tablets, granules, pellets, capsules.

As used herein, the term "metformin" includes metformin and pharmaceutically acceptable salts thereof. The pharmaceutically acceptable salts include hydrochloride, hydrobromide, fumarate, malonate, malate, succinate, lactate, glycolate, maleate, citrate, and aspartate. The pharmaceutically acceptable salts of metformin may be present in hydrous or anhydrous forms. They may also be present in crystalline or amorphous forms. In this invention, preferably metformin hydrochloride is used.

As used herein, the term "sitagliptin" includes sitagliptin and pharmaceutically acceptable salts thereof. The pharmaceutically acceptable salts include hydrochloride, hydrobromide, phosphate, sulphate, mesylate, besylate, tosylate, fumarate, malonate, malate, succinate, lactate, glycolate, maleate, citrate, and aspartate. The pharmaceutically acceptable salts of sitagliptin may be present in hydrous or anhydrous forms. The hydrate forms may be monohydrate or dihydrate forms. The pharmaceutically acceptable salts of sitagliptin may also be present in crystalline or amorphous forms. In this invention, preferably sitagliptin malate is used.

Sitagliptin and metformin combination has incompatibility and stability problems due to interactions between drug substances. Separating metformin and sitagliptin in such a way eliminates their interaction in an effective way so as to overcome these problems.

In the present invention, multi-layered tablet is designed by placing these two drug substances in different layers that solve the interaction problems. Furthermore, this is an easy and cost-effective process and provides improvement in dissolution profile.

Multi-layered tablets are known as a novel drug delivery system. Compaction of different granules in the form of various layers in single tablets is called as multi-layered tablets. It generally consists of parallel, clear, coloured, visual distinct layers two to three or more APIs or APIs along with functional or non-functional layers. Multi-layered tablet dosage forms are designed for a variety of reasons such as incompatibility problems between active agents or excipients.

In accordance with the present invention, a pharmaceutical formulation is provided, as defined by the claims, which is in the form of multi-layered tablet which comprising metformin and sitagliptin. Such a combination provides desired efficacy while minimizing problem of incompatibility.

According to the present invention, the pharmaceutical tablet formulation comprises;
a) a core having metformin and at least one dispersion carriers
b) the first layer
c) the second layer having sitagliptin
wherein the core and the second layer are separated by the first layer, as defined by the claims. So, the first layer acts as a barrier for interactions and this overcomes the stability problem caused by the incompatibility of metformin and sitagliptin.

In the present invention, the first layer does not comprise an active pharmaceutical agent and that typically will rapidly disperse or dissolves in water.

According to another embodiment of the present invention, the total composition comprises a core, the first layer, the second layer.

According to one embodiment of the present invention, the weight ratio of the core to second layer is 1.0-25.0, preferably the ratio is 1.0-23.0, more preferably 1.0-22.0. This ratio provides desired dissolution profile of active agents and desired efficacy.

According to one embodiment of the present invention, the amount of the core in the tablet is between 70.0% and 97.0%, between 85.0% and 93.0% by weight of the total composition.

According to one embodiment of the present invention, the amount of the second layer in the tablet is between 2.0% and 8.0% by weight of the total composition.

Also, as known, metformin is a very poorly compressible active substance and in the present invention, metformin presents in high amounts in the tablet. It was surprisingly found that hot melt extruded metformin is compressed into the core without the need of wet granulation or addition of too many excipients. Also, this process provides content uniformity and enhanced dissolution profile. Furthermore, this process not only prevents decomposition of metformin in the process but also recrystallization during storage,because no liquid solvent is used during hot melt extrusion.

According to one embodiment of the present invention, said formulation is obtained by means of a hot-melt extrusion not involving any liquid solvent.

Hot-melt extrusion (HME) technology is prominent in the pharmaceutical industry. HME offers the potential of shorter and more efficient times to the final product, through reduction of the processing steps involved. HME is used to disperse active agent in a matrix at the molecular level, thus forming solid -dispersions. This method is used for poorly soluble active agents and so desired dissolution rate and stability are obtained.

In the present invention, the core comprises extrudate which is a solid dispersion of metformin and at least one dispersion carrier prepared by the process of hot melt extrusion. The process does not comprise liquid solvent and this contributes to stability of metformin.

Suitable dispersion carriers are selected from polymers or non-polymers. Suitable polymers are selected from the group comprising hydroxypropyl cellulose (HPC), hydroxypropyl methylcellulose and their derivatives, polyvinylpyrrolidone, copolymer of ethyl acrylate or methyl methacrylate (Eudragit L100 55), polyvinyl acetal diethylaminoacetate, polyvinyl caprolactam-polyvinyl acetate-polyethylene glycol graft copolymer, polyoxyl 40 hydrogenated castor oil, hydrogenated castor oil, polyoxyl 15 hydroxystearate, polyoxyl castor oil, polyvinyl alcohol/polyethylene glycol graft copolymer, cetearyl ethyl hexanone/isopropyl myristate, gliceryl monostearate, polyethylene glycol or mixtures thereof.

According to one embodiment of the present invention, the dispersion carrier is HPC, in other words, hydroxypropyl cellulose which is a thermoplastic polymer.

According to one embodiment of the present invention, the amount of HPC in the tablet is between 13.0% and 44.0%, between 15.0% and 42.0%, between 20.0% and 40.0% by weight of the total composition.

According to one embodiment of the present invention, metformin is compounded with HPC, which acts as a solid solvent, and extruded at an appropriate temperature with shear stress to form solid dispersion. It has been surprisingly found that hot melt extruded dosage form of metformin with the polymer HPC achieves desired solubility with physical and chemical stability of the core in the tablet.

According to the present invention, the ratio of metformin to HPC is in the range of 3:1 and 1:3 by weight. This ratio is important in order to prepare a successful extrudate ensuring desired solubility and homogeneity.

According to one embodiment of the present invention, the formulation further comprises pharmaceutically acceptable excipients which are selected from disintegrants, fillers, binders, surfactants, glidants, lubricants, coating agents, coloring agents, solvents or mixtures thereof.

Suitable disintegrants are selected from the group comprising sodium starch glycolate, low-substituted hydroxypropyl cellulose, cross-linked polyvinylpyrrolidone, cross-linked sodium carboxymethylcellulose, alginates, gums, cross-linked calcium carboxymethylcellulose, sodium carboxymethylcellulose, ion-exchange resins or mixtures thereof.

According to one embodiment of the present invention, the amount of disintegrants in the tablet is between 0.01% and 3.0%, preferably it is between 0.09% and 2.0% by weight of the total composition.

According to one embodiment of the present invention, preferably the disintegrant is sodium starch glycolate.

Suitable fillers and binders are selected from group comprising microcrystalline cellulose, lactose monohydrate, starch, mannitol, dibasic calcium phosphate, tribasic calcium phosphate, trehalose, isomalt, sodium carbonate, sodium bicarbonate, calcium carbonate, carboxymethyl cellulose, polydextrose, polyethylene oxide, hydroxypropyl methyl cellulose, methyl cellulose, polyethylene glycol or mixtures thereof.

According to one embodiment of the present invention, the amount of fillers and binders in the tablet is between 1.0% and 10.0%, preferably it is between 2.0% and 8.5% by weight of the total composition.

According to one embodiment of the present invention, preferably the filler and binder is microcrystalline cellulose.

Suitable surfactants are selected from the group comprising polyoxyethylene sorbitan esters (polysorbate), propylene glycol, glyceryl oleate, tocopherol, ascorbyl palmitate, citric acid, polyethoxylated fatty acid esters, polyoxyethylene hydrogenated castor oil, sorbitan esters, sodium lauryl sulphate, docusate sodium, nonoxynol or mixtures thereof.

According to one embodiment of the present invention, the amounts of surfactants in the tablet is between 0.0001% and 4.0%, preferably it is between 0.0001% and 2.5% by weight of the total composition.

According to one embodiment of the present invention, preferably the surfactant is polyoxyethylene sorbitan esters (polysorbate).

Hot-melt extrusion is a technique for manufacturing amorphous solid dispersions in which the active agent and dispersion carrier are fed into extruder and exposed to shear stress at an appropriate temperature. The extrudate obtained is then compressed into tablet with functional excipients, such as glidants or lubricants, with the purpose of processability.

Suitable glidants or lubricants are selected from group comprising magnesium stearate, colloidal silicon dioxide, talc, sodium stearyl fumarate, polyethylene glycol, sodium lauryl sulphate, magnesium lauryl sulphate, fumaric acid, glyceryl palmitostearate, hydrogenated natural oils, zinc stearate, calcium stearate, silica, stearic acid, polyethylene glycol, paraffin or mixtures thereof.

According to one embodiment of the present invention, the amount of glidants or lubricants in the tablet is between 0.1% and 5.0%, preferably it is between 0.5% and 4.0% by weight of the total composition.

According to one embodiment of the present invention, preferably the glidant or lubricant is magnesium stearate or colloidal silicon dioxide or talc or mixtures thereof.

According to one embodiment of the present invention, the formulation further comprises the third layer having at least one coating agent.

According to another embodiment of the present invention, the pharmaceutical tablet formulation comprises;
a) a core having metformin and at least one dispersion carriers
b) the first layer
c) the second layer having sitagliptin
d) the third layer having at least one coating agent
wherein the core and the second layer are separated by the first layer.

According to another embodiment of the present invention, the first layer and the third layer do not comprise an active pharmaceutical agent and that typically will rapidly disperse or dissolves in water.

Suitable coating agents are selected from the group comprising polyvinyl alcohol (PVA), titanium dioxide, polyethylene glycol (PEG), talc, hydroxypropyl methylcellulose, polymethacrylates, triacetin, glycerol triacetin, lactose monohydrate, hydroxypropyl cellulose, polyvinyl alcohol-polyethylene glycol copolymers (Kollicoat^{®} IR), ethylcellulose dispersions (Surelease^{®}), polyvinylpyrrolidone, polyvinylpyrrolidone-vinyl acetate copolymer (PVP-VA), all kinds of Opadry^{®}, macrogol, coloring agents or mixtures thereof.

Suitable coloring agents are selected from the group comprising ferric oxide, Food, Drug & Cosmetic (FD&C) dyes (such as; FD&C blue, FD&C green, FD&C red, FD&C yellow, FD&C lakes), ponceau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow or mixtures thereof.

Preferably, coating agents are polyvinyl alcohol (PVA), titanium dioxide, polyethylene glycol (PEG), talc or mixtures thereof.

The pharmaceutical multi-layer tablet of the present invention may be prepared by using standard techniques and manufacturing processes well known in the art, such as coating with active substance and compression. The layers of the tablet may be manufactured with conventional methods including wet or dry granulation, hot melt extrusion, fluidized bed granulation etc. Preferably the core is compressed with hot melt extruded granule and other layers are prepared either with compression or coating.

During wet granulation process, a liquid solvent is used. Suitable liquid solvents are selected from the group comprising pure water, isopropyl alcohol, propylene glycol, polyethylene glycol, glycerin, ethanol or mixtures thereof.

According to one embodiment of the present invention, preferably the liquid solvent is pure water or isopropyl alcohol or mixtures thereof.

According to one embodiment of the present invention, the below-described heating temperatures are used at zones during the hot melt extrusion. The temperature is chosen according to the properties of the dispersion carriers and metformin.

Preferably heating is adjusted to a temperature which is above the glass transition temperature of the dispersion carrier and sometimes above its melting temperature to achieve molecular level mixing of metformin and dispersion carrier.

Preferably the heating temperature is chosen between 35°C and 190°C, preferably between 40°C and 185°C.

The temperature ranges given above is suitable at this invention to provide high solubility of metformin, high physical and chemical stability.

According to another embodiment of the present invention, the total composition comprises a core, the first layer, the second layer and the third layer.

According to one embodiment of the present invention, the core comprises 35.0% - 72.0% by weight of metformin, 13.0% - 44.0% by weight of hydroxypropyl cellulose (HPC), 0.1% - 3.0% by weight of colloidal silicon dioxide, 0.1% - 3.0% by weight of magnesium stearate by weight of the total composition.

According to one embodiment of the present invention, extrudate obtained by hot melt extrusion process comprises metformin and at least one dispersion carrier.

In other words, the core comprises 60.0% - 95.0%by weight of extrudate and 0.1% - 3.0% by weight of colloidal silicon dioxide, 0.1% - 3.0% by weight of magnesium stearate by weight of the total composition.

According to one embodiment of the present invention, the first layer comprises 0.1% - 3.0% by weight of coating agents, 0.001% - 8.0% by weight of pure water by weight of the total composition.

According to another embodiment of the present invention, the first layer comprises 0.1% - 3.0% by weight of colloidal silicon dioxide, 1.0% - 8.0% by weight of microcrystalline cellulose, 0.1% - 3.0% by weight of magnesium stearate by weight of the total composition.

According to one embodiment of the present invention, the first layer may be a coating over the core or it may be compressed as a layer on the core.

According to one embodiment of the present invention, the second layer comprises 1.0% - 10.0% by weight of sitagliptin, 0.0001% - 4.0% by weight of polysorbate, 0.01% - 3.0% by weight of hydroxypropyl cellulose, 0.001% - 2.0% by weight of isopropyl alcohol, 0.001% - 2.0% by weight of pure water by weight of the total composition.

According to another embodiment of the present invention, the second layer comprises 1.0% - 10.0% by weight of sitagliptin, 0.01% - 3.0% by weight of sodium starch glycolate, 0.1% - 4.0% by weight of microcrystalline cellulose, 0.01% - 2.0% by weight of magnesium stearate, 0.01% - 2.0% by weight of talc and quantity sufficient of pure water by weight of the total composition.

According to one embodiment of the present invention, the second layer may be a coating over the first layer or it may be compressed as a layer on the first layer.

According to one embodiment of the present invention, the third layer comprises 0.1% - 3.0% by weight of coating agents and quantity sufficient of pure water by weight of the total composition.

According to one embodiment of the present invention, the third layer may be a coating over the second layer or it may be compressed as a layer on the second layer.

### Example 1

| | **(%) amount (w/w)** |
|---|---|
| **Core** | **70.0 - 97.0** |
| Metformin | 35.0 - 72.0 |
| Hydroxypropyl cellulose (HPC) | 13.0 - 44.0 |
| Colloidal silicon dioxide | 0.1 - 3.0 |
| Magnesium stearate | 0.1 - 3.0 |
| **First layer** | **0.5 - 3.0** |
| Coating agents | 0.1 - 3.0 |
| Pure water | 0.001 - 8.0 |
| **Second layer** | **1.0 - 11.0** |
| Sitagliptin | 1.0 - 10.0 |
| Polysorbate | 0.0001 - 4.0 |
| Hydroxypropyl cellulose | 0.01 - 3.0 |
| Isopropyl alcohol | 0.001 - 2.0 |
| Pure water | 0.001 - 2.0 |
| **Third layer** | **0.5 - 3.0** |
| Coating agents | 0.1 - 3.0 |
| Pure water | q.s. |
| **Total composition** | **100** |

| | |
|---|---|
| q.s.: quantity sufficient | |

Process for the preparation of the tablet according to example 1,
**Core**
   a) Mixing metformin and HPC until a homogeneous mixture is obtained,
   b) Extruding the mixture prepared at step(a) at an appropriate temperature with shear stress,
   c) Cooling the extrudate obtained at step(b)
   d) Then, sieving the extrudate
   e) Adding colloidal silicon dioxide and mixing
   f) Finally, adding magnesium stearate and mixing
   g) Compressing the mixture to form core
**First layer**
   h) Preparing coating solution with coating agents and pure water
   i) Coating the core with at step (h) coating solution
**Second layer**
   j) Preparing solution with isopropyl alcohol:pure water
   k) Adding hydroxypropyl cellulose, polysorbate and sitagliptin in step (j) solution
   l) Coating the first layer with at step (k) solution
**Third layer**
   m) Preparing coating solution with coating agents and pure water
   n) Coating the second layer with at step (m) coating solution

### Example 2

| | **(%) amount (w/w)** |
|---|---|
| **Core** | **70.0 - 97.0** |
| Metformin | 35.0 - 72.0 |
| Hydroxypropyl cellulose (HPC) | 13.0 - 44.0 |
| Colloidal silicon dioxide | 0.1 - 3.0 |
| Magnesium stearate | 0.1 - 3.0 |
| **First layer** | **0.5 - 3.0** |
| Colloidal silicon dioxide | 0.1 - 3.0 |
| Microcrystalline cellulose | 1.0 - 8.0 |
| Magnesium stearate | 0.1 - 3.0 |
| **Second layer** | **1.0 - 11.0** |
| Sitagliptin | 1.0 - 10.0 |
| Sodium starch glycolate | 0.01 - 3.0 |
| Microcrystalline cellulose | 0.1 - 4.0 |
| Magnesium stearate | 0.01 - 2.0 |
| Talc | 0.01 - 2.0 |
| Pure water | q.s. |
| **Third layer** | **0.5 - 3.0** |
| Coating agents | 0.1 - 3.0 |
| Pure water | q.s. |
| **Total composition** | **100** |
| q.s.: quantity sufficient | |

Process for the preparation of the tablet according to example 2,
**Core**
   a) Mixing metformin and HPC until a homogeneous mixture is obtained,
   b) Extruding the mixture prepared at step (a) at an appropriate temperature with shear stress,
   c) Cooling the extrudate obtained at step (b)
   d) Then, sieving the extrudate
   e) Adding colloidal silicon dioxide and mixing
   f) Finally, adding magnesium stearate and mixing
   g) Compressing the mixture to form core
**First layer**
   h) Mixing colloidal silicon dioxide, microcrystalline cellulose and magnesium stearate
   i) Pressing the mixture prepared at the step (o) as the first layer onto the core
**Second layer**
   j) Mixing sitagliptin, sodium starch glycolate, microcrystalline cellulose
   k) Adding pure water and granulating then, drying, sieving
   l) Adding magnesium stearate and talc
   m) Compressing the mixture prepared at the step (s) as the second layer onto the first layer
**Third layer**
   n) Preparing coating solution with coating agents and pure water
   o) Compressing coating solution prepared at the step (u) as the third layer onto the multi-layered tablet

## Claims

1. A pharmaceutical tablet formulation comprising;
a) a core having metformin and at least one dispersion carriers
b) the first layer
c) the second layer having sitagliptin
wherein the core and the second layer are separated by the first layer and wherein the amount of the second layer in the tablet is between 2.0% and 8.0% by weight of the total composition and wherein
- the dispersion carrier is hydroxypropyl cellulose,
- the ratio of metformin to hydroxypropyl cellulose is in the range of between 3:1 and 1:3 by weight, and
- the pharmaceutical tablet formulation is obtained by hot melt extrusion.

2. The pharmaceutical tablet formulation according to claim 1, wherein the weight ratio of the core to the second layer is 1.0 - 25.0.

3. The pharmaceutical tablet formulation according to claim 1, wherein the amount of the core in the tablet is between 70.0% and 97.0% by weight of the total composition.

4. The pharmaceutical tablet formulation according to claim 1, wherein said formulation is obtained by means of a hot-melt extrusion not involving any liquid solvent.

5. The pharmaceutical tablet formulation according to claim 1, wherein the amount of hydroxypropyl cellulose in the tablet is between 13.0% and 44.0%, preferably it is between 15.0% and 42.0%.

6. The pharmaceutical tablet formulation according to claim 1, further comprising pharmaceutically acceptable excipients which are selected from disintegrants, fillers, binders, surfactants, glidants, lubricants, coating agents, coloring agents, solvents or mixtures thereof.

7. The pharmaceutical tablet formulation according to claim 6, wherein the glidants or lubricants are selected from group comprising magnesium stearate, colloidal silicon dioxide, talc, sodium stearyl fumarate, polyethylene glycol, sodium lauryl sulphate, magnesium lauryl sulphate, fumaric acid, glyceryl palmitostearate, hydrogenated natural oils, zinc stearate, calcium stearate, silica, stearic acid, polyethylene glycol, paraffin or mixtures thereof.

8. The pharmaceutical tablet formulation according to claim 1, further comprising the third layer having at least one coating agent.

9. The pharmaceutical tablet formulation according to claim 8, wherein the formulation comprising;
a) a core having metformin and at least one dispersion carriers
b) the first layer
c) the second layer having sitagliptin
d) the third layer having at least one coating agent

10. The pharmaceutical tablet formulation according to any preceding claims, wherein the formulation comprising;
A. Core
35.0% - 72.0% by weight of metformin
13.0% - 44.0% by weight of hydroxypropyl cellulose
0.1% - 3.0% by weight of colloidal silicon dioxide
0.1% - 3.0% by weight of magnesium stearate by weight of the total composition.
B. First layer
0.1% - 3.0% by weight of coating agents
0.001% - 8.0% by weight of pure water by weight of the total composition.
C. Second layer
1.0% - 10.0% by weight of sitagliptin
0.0001% - 4.0% by weight of polysorbate
0.01% - 3.0% by weight of hydroxypropyl cellulose
0.001% - 2.0% by weight of isopropyl alcohol
0.001% - 2.0% by weight of pure water by weight of the total composition.
D. Third layer
quantity sufficient of pure water
0.1% - 3.0% by weight of coating agents by weight of the total composition.

11. The pharmaceutical tablet formulation according to any preceding claims, wherein the formulation comprising;
A. Core
35.0% - 72.0% by weight of metformin
13.0% - 44.0% by weight of hydroxypropyl cellulose
0.1% - 3.0% by weight of colloidal silicon dioxide
0.1% - 3.0% by weight of magnesium stearate by weight of the total composition.
B. First layer
0.1% - 3.0% by weight of colloidal silicon dioxide
1.0% - 8.0% by weight of microcrystalline cellulose
0.1% - 3.0% by weight of magnesium stearate by weight of the total composition.
C. Second layer
1.0% - 10.0% by weight of sitagliptin
0.01% - 3.0% by weight of sodium starch glycolate
0.1% - 4.0% by weight of microcrystalline cellulose
0.01% - 2.0% by weight of magnesium stearate
quantity sufficient of pure water
0.01% - 2.0% by weight of talc by weight of the total composition.
D. Third layer
quantity sufficient of pure water
0.1% - 3.0% by weight of coating agents by weight of the total composition.

## Patentansprüche

1. Eine pharmazeutische Tablettenformulierung, umfassend:
a) einen Kern mit Metformin und mindestens einem Dispersionssträger
b) die erste Schicht
c) die zweite Schicht mit Sitagliptin
wobei der Kern und die zweite Schicht durch die erste Schicht getrennt sind und wobei die Menge der zweiten Schicht in der Tablette zwischen 2,0 Gew.-% und 8,0 Gew.-% der Gesamtzusammensetzung liegt und wobei
- der Dispersionssträger Hydroxypropylcellulose ist,
- das Gewichtsverhältnis von Metformin zu Hydroxypropylcellulose im Bereich zwischen 3:1 und 1:3 liegt, und
- die pharmazeutische Tablettenformulierung durch Schmelzextrusion (Hot-Melt-Extrusion) erhalten wird.

2. Die pharmazeutische Tablettenformulierung nach Anspruch 1, wobei das Gewichtsverhältnis des Kerns zur zweiten Schicht 1,0 bis 25,0 beträgt.

3. Die pharmazeutische Tablettenformulierung nach Anspruch 1, wobei die Menge des Kerns in der Tablette zwischen 70,0 Gew.-% und 97,0 Gew.-% der Gesamtzusammensetzung liegt.

4. Die pharmazeutische Tablettenformulierung nach Anspruch 1, wobei die Formulierung mittels einer Schmelzextrusion erhalten wird, die kein flüssiges Lösungsmittel beinhaltet.

5. Die pharmazeutische Tablettenformulierung nach Anspruch 1, wobei die Menge an Hydroxypropylcellulose in der Tablette zwischen 13,0 Gew.-% und 44,0 Gew.-% liegt, vorzugsweise liegt sie zwischen 15,0 Gew.-% und 42,0 Gew.-%.

6. Die pharmazeutische Tablettenformulierung nach Anspruch 1, ferner umfassend pharmazeutisch annehmbare Hilfsstoffe, die ausgewählt sind aus Sprengmitteln, Füllstoffen, Bindemitteln, tensidischen Stoffen (Mittel zur Verringerung der Oberflächenspannung), Fließregulierungsmitteln, Schmiermitteln, Überzugsmitteln, Farbstoffen, Lösungsmitteln oder Mischungen davon.

7. Die pharmazeutische Tablettenformulierung nach Anspruch 6, wobei die Fließregulierungsmittel oder Schmiermittel ausgewählt sind aus der Gruppe umfassend Magnesiumstearat, kolloidales Siliciumdioxid, Talkum, Natriumstearylfumarat, Polyethylenglycol, Natriumlaurylsulfat, Magnesiumlaurylsulfat, Fumarsäure, Glycerylpalmitostearat, hydrierte natürliche Öle, Zinkstearat, Calciumstearat, Kieselsäure, Stearinsäure, Polyethylenglycol, Paraffin oder Mischungen davon.

8. Die pharmazeutische Tablettenformulierung nach Anspruch 1, ferner umfassend die dritte Schicht mit mindestens einem Überzugsmittel.

9. Die pharmazeutische Tablettenformulierung nach Anspruch 8, wobei die Formulierung umfasst:
a) einen Kern mit Metformin und mindestens einem Dispersionssträger
b) die erste Schicht
c) die zweite Schicht mit Sitagliptin
d) die dritte Schicht mit mindestens einem Überzugsmittel

10. Die pharmazeutische Tablettenformulierung nach einem der vorhergehenden Ansprüche, wobei die Formulierung umfasst:
A. Kern
35,0 Gew.-% - 72,0 Gew.-% Metformin
13,0 Gew.-% - 44,0 Gew.-% Hydroxypropylcellulose
0,1 Gew.-% - 3,0 Gew.-% kolloidales Siliciumdioxid
0,1 Gew.-% - 3,0 Gew.-% Magnesiumstearat, bezogen auf das Gewicht der Gesamtzusammensetzung.
B. Erste Schicht
0,1 Gew.-% - 3,0 Gew.-% Überzugsmittel
0,001 Gew.-% - 8,0 Gew.-% reines Wasser, bezogen auf das Gewicht der Gesamtzusammensetzung.
C. Zweite Schicht
1,0 Gew.-% - 10,0 Gew.-% Sitagliptin
0,0001 Gew.-% - 4,0 Gew.-% Polysorbat
0,01 Gew.-% - 3,0 Gew.-% Hydroxypropylcellulose
0,001 Gew.-% - 2,0 Gew.-% Isopropanol (Isopropylalkohol)
0,001 Gew.-% - 2,0 Gew.-% reines Wasser, bezogen auf das Gewicht der Gesamtzusammensetzung.
D. Dritte Schicht
eine ausreichende Menge an reinem Wasser
0,1 Gew.-% - 3,0 Gew.-% Überzugsmittel, bezogen auf das Gewicht der Gesamtzusammensetzung.

11. Die pharmazeutische Tablettenformulierung nach einem der vorhergehenden Ansprüche, wobei die Formulierung umfasst:
A. Kern
35,0 Gew.-% - 72,0 Gew.-% Metformin
13,0 Gew.-% - 44,0 Gew.-% Hydroxypropylcellulose
0,1 Gew.-% - 3,0 Gew.-% kolloidales Siliciumdioxid
0,1 Gew.-% - 3,0 Gew.-% Magnesiumstearat, bezogen auf das Gewicht der Gesamtzusammensetzung.
B. Erste Schicht
0,1 Gew.-% - 3,0 Gew.-% kolloidales Siliciumdioxid
1,0 Gew.-% - 8,0 Gew.-% mikrokristalline Cellulose
0,1 Gew.-% - 3,0 Gew.-% Magnesiumstearat, bezogen auf das Gewicht der Gesamtzusammensetzung.
C. Zweite Schicht
1,0 Gew.-% - 10,0 Gew.-% Sitagliptin
0,01 Gew.-% - 3,0 Gew.-% Natriumstärke-Glykolat
0,1 Gew.-% - 4,0 Gew.-% mikrokristalline Cellulose
0,01 Gew.-% - 2,0 Gew.-% Magnesiumstearat
eine ausreichende Menge an reinem Wasser
0,01 Gew.-% - 2,0 Gew.-% Talkum, bezogen auf das Gewicht der Gesamtzusammensetzung.
D. Dritte Schicht
eine ausreichende Menge an reinem Wasser
0,1 Gew.-% - 3,0 Gew.-% Überzugsmittel, bezogen auf das Gewicht der Gesamtzusammensetzung.

## Revendications

1. Formulation de comprimé pharmaceutique comprenant ;
a) un noyau comportant de la metformine et au moins un support de dispersion
b) la première couche
c) la deuxième couche comportant de la sitagliptine
dans laquelle le noyau et la deuxième couche sont séparés par la première couche et dans laquelle la quantité de la deuxième couche dans le comprimé est comprise entre 2,0 % et 8,0 % en poids de la composition totale, et dans laquelle
- le support de dispersion est l'hydroxypropylcellulose,
- le rapport entre la metformine et l'hydroxypropylcellulose se situe dans la plage allant de 3:1 et 1:3 en poids, et
- la formulation de comprimé pharmaceutique est obtenue par extrusion à chaud.

2. Formulation de comprimé pharmaceutique selon la revendication 1, dans laquelle le rapport pondéral du noyau à la deuxième couche est de 1,0 - 25,0.

3. Formulation de comprimé pharmaceutique selon la revendication 1, dans laquelle la quantité du noyau dans le comprimé est comprise entre 70,0 % et 97,0 % en poids de la composition totale.

4. Formulation de comprimé pharmaceutique selon la revendication 1, dans laquelle ladite formulation est obtenue au moyen d'une extrusion à chaud ne faisant intervenir aucun solvant liquide.

5. Formulation de comprimé pharmaceutique selon la revendication 1, dans laquelle la quantité d'hydroxypropylcellulose dans le comprimé est comprise entre 13,0 % et 44,0 %, de préférence entre 15,0 % et 42,0 %.

6. Formulation de comprimé pharmaceutique selon la revendication 1, comprenant en outre des excipients pharmaceutiquement acceptables qui sont choisis parmi les désintégrants, les charges, les liants, les tensioactifs, les agents d'écoulement, les lubrifiants, les agents d'enrobage, les colorants, les solvants ou leurs mélanges.

7. Formulation de comprimé pharmaceutique selon la revendication 6, dans laquelle les agents d'écoulement ou les lubrifiants sont choisis dans le groupe comprenant le stéarate de magnésium, le dioxyde de silicium colloïdal, le talc, le stéarylfumarate de sodium, le polyéthylèneglycol, le laurylsulfate de sodium, le laurylsulfate de magnésium, l'acide fumarique, le palmitostéarate de glycéryle, les huiles naturelles hydrogénées, le stéarate de zinc, le stéarate de calcium, la silice, l'acide stéarique, la paraffine ou leurs mélanges.

8. Formulation de comprimé pharmaceutique selon la revendication 1, comprenant en outre la troisième couche comportant au moins un agent d'enrobage.

9. Formulation de comprimé pharmaceutique selon la revendication 8, dans laquelle la formulation comprenant ;
a) un noyau comportant de la metformine et au moins un support de dispersion ;
b) la première couche ;
c) la deuxième couche comportant de la sitagliptine ;
d) la troisième couche comportant au moins un agent d'enrobage.

10. Formulation de comprimé pharmaceutique selon l'une quelconque des revendications précédentes, dans laquelle la formulation comprenant ;
A. Noyau
35,0 % - 72,0 % en poids de metformine
13,0 % - 44,0 % en poids d'hydroxypropylcellulose
0,1 % - 3,0 % en poids de dioxyde de silicium colloïdal
0,1 % - 3,0 % en poids de stéarate de magnésium, en poids de la composition totale
B. Première couche
0,1 % - 3,0 % en poids d'agents d'enrobage
0,001 % - 8,0 % en poids d'eau purifiée, en poids de la composition totale.
C. Deuxième couche
1,0 % - 10,0 % en poids de sitagliptine
0,0001 % - 4,0 % en poids de polysorbate
0,01 % - 3,0 % en poids d'hydroxypropylcellulose
0,001 % - 2,0 % en poids d'alcool isopropylique
0,001 % - 2,0 % en poids d'eau purifiée, en poids de la composition totale.
D. Troisième couche
quantité suffisante d'eau purifiée
0,1 % - 3,0 % en poids d'agents d'enrobage, en poids de la composition totale.

11. Formulation de comprimé pharmaceutique selon l'une quelconque des revendications précédentes, dans laquelle la formulation comprenant ;
A. Noyau
35,0 % - 72,0 % en poids de metformine
13,0 % - 44,0 % en poids d'hydroxypropylcellulose
0,1 % - 3,0 % en poids de dioxyde de silicium colloïdal
0,1 % - 3,0 % en poids de stéarate de magnésium, en poids de la composition totale.
B. Première couche
0,1 % - 3,0 % en poids de dioxyde de silicium colloïdal
1,0 % - 8,0 % en poids de cellulose microcristalline
0,1 % - 3,0 % en poids de stéarate de magnésium, en poids de la composition totale.
C. Deuxième couche
1,0 % - 10,0 % en poids de sitagliptine
0,01 % - 3,0 % en poids de glycolate d'amidon sodique
0,1 % - 4,0 % en poids de cellulose microcristalline
0,01 % - 2,0 % en poids de stéarate de magnésium quantité suffisante d'eau purifiée
0,01 % - 2,0 % en poids de talc, en poids de la composition totale.
D. Troisième couche
quantité suffisante d'eau purifiée
0,1 % - 3,0 % en poids d'agents d'enrobage, en poids de la composition totale.
